# EUROPEAN PATENT APPLICATION

(11) **EP 2 692 854 A1**
(43) Date of publication of application: **05.02.2014**
(21) Application number: 12764708.9
(22) Date of filing: 28.03.2012
(51) Int. Cl.: C12M 3/00

(54) **CULTURE VESSEL FOR FORMING EMBRYOID BODY**

(30) Priority: 30.03.2011 JP 2011076621
(71) Applicant: Sumitomo Bakelite Co., Ltd., Shinagawa-ku Tokyo 140-0002 (JP)
(72) Inventor: SAKURA Takeshi, Tokyo 140-0002 (JP); TSUKADA Ryouhei, Tokyo 140-0002 (JP)
(74) Representative: Ahner, Philippe
(86) International application number: PCT/JP2012/058103
(87) International publication number: WO 2012/133514

(57) **Abstract**

The present invention provides a culture vessel for forming an embryoid body that makes it possible to form an embryoid body of higher quality, more efficiently, at a higher level. The present invention relates to a culture vessel for forming an embryoid body that has two or more wells, in which the wells have a bottom having a cross section that looks like approximately a U shape when being observed in a vertical direction and an opening having an approximately circular shape, at least a curved portion of the inner surface of the bottom has low cell adhesive properties, a curvature radius (R') of the inner surface of the bottom is from 1.0 mm to 3.5 mm, and the low cell adhesion surface is formed by being performed low-or-non cell adhesion treatment with a water-soluble resin.

## Description

### Technical Field

The present invention relates to a culture vessel for forming an embryoid body.

Priority is claimed on Japanese Patent Application No. 2011-76621, filed March 30, 2011, the content of which is incorporated herein by reference.

### Background Art

An embryonic stem cell (ES cell) is obtained by seeding the inner cell mass of a blastocyst on a feeder cell, adding a leukemia inhibitory factor (LIF) thereto, followed by culturing.

The ES cell that infinitely proliferates while maintaining of an undifferentiated state has pluripotency by which the cell is differentiated into various tissue cells. Accordingly, the ES cell has become an object of diverse study in the field of transplantation therapy using tissues obtained by differentiation, that is, in the field of so-called regenerative medicine.

In order to induce the ES cell to be differentiated into various tissues, a method of forming a pseudo-embryo called an embryoid body (EB) is the most widely used, and forming an EB is the first step in the induction of differentiation of the ES cell *in vitro.* Moreover, for forming the EB, it is necessary to culture the ES cell in a state where the ES cell is floating without adhering to a culture vessel. In adhesion culture using a general culture vessel, the EB is not formed, and the ES cell undergoes adhesion and extension and starts to be non-specifically differentiated.

For culturing the ES cell in a floating state, a method called hanging-drop culture is the most widely used. Like its name, the hanging-drop culture is a method of culturing cells in culture fluid hung in the shape of water drops. That is, mineral oil and a buffer solution are added to wells of a multi-well plate, a culture suspension containing the ES cell is spotted in the shape of liquid drops to the positions of the lid of the multi-well plate that overlap the respective wells, and the lid is put on the multi-well plate to culture the cell. However, the hanging-drop method has problems in that a success rate of EB formation is low, microscopic observation is unavailable, the amount of EB that can be formed at a time is small, and the operation is complicated, and the like.

In order to solve the above problems, Patent Document 1 suggests a production method of a culture vessel for forming an embryoid body that includes a step of forming a water-soluble resin-covering layer in which the inner surface of a culture vessel is covered with a water-soluble resin to form a water-soluble covering layer and a step of modifying into a water-insoluble cured film which is performed after the above step and in which the water-soluble covering layer is cured so as to be modified into a water-insoluble cured film layer, a culture vessel for forming an embryoid body that is produced by the production method, and use of the vessel.

### Prior Art Documents

### Patent Documents

Patent Document 1: Japanese Unexamined Patent Application, First Publication No. 2008-178367

### Disclosure of the Invention

### Problems to be Solved by the Invention

However, in view of efficiently forming a high-quality embryoid body from the ES cell, the technique disclosed in Patent Document 1 can be further improved.

Therefore, the present invention aims to provide a culture vessel for forming an embryoid body that makes it possible to efficiently form a high-quality embryoid body at a higher level.

### Means for Solving the Problems

The present inventors conducted thorough research, and as a result, they found that if the inner surface of each well in a culture vessel having two or more wells is performed low-or-non cell adhesion treatment with a water-soluble resin and is made into a predetermined shape, a cell aggregate solvent can be generated, and consequently, the above object can be achieved. The present invention has been completed in this manner.

That is, the above object can be achieved by the present invention described in the following (1) to (4).
(1) A culture vessel for forming an embryoid body that has two or more wells, wherein the wells have a bottom having a cross section that looks like an approximately U shape when being observed in a vertical direction and an opening having an approximately circular shape, at least a curved portion of an inner surface of the bottom has low cell adhesive properties, a curvature radius (R') of the inner surface of the bottom is from 1.0 mm to 3.5 mm, and the low cell adhesion surface is formed by being performed low-or-non cell adhesion treatment with a water-soluble resin.
(2) The culture vessel for forming an embryoid body according to (1), wherein the water-soluble resin is a compound represented by the following Formula (Ia) or (Ib). 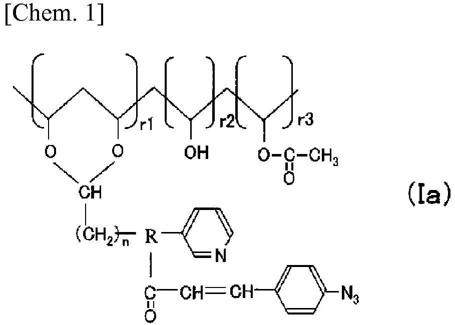
r1 = 1 to 1000, r2 = 40 to 4995, r3 = 0 to 4000, n = 1, 2 or 3, and R represents an alkyl group having carbonyl and amine. 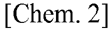 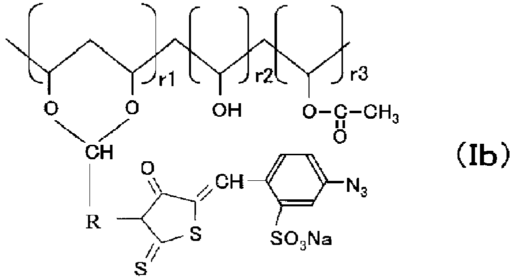 r1 = 1 to 1000, r2 = 40 to 4995, r3 = 0 to 4000, and R represents an alkyl group having carbonyl and amine.

(3) The culture vessel for forming an embryoid body according to (1) or (2), wherein at least one dent having a low cell adhesion surface is provided inside the surface of the lowermost portion of the well.
(4) The culture vessel for forming an embryoid body according to any one of (1) to (3), wherein an entire inner surface of each well has low cell adhesive properties.

According to the present invention, it is possible to form a cell aggregate of higher quality, more efficiently, at a higher level.

### Brief Description of the Drawings

FIG. 1 shows the cross-sectional shape of a culture well.
FIG. 2 is a view schematically showing a state where a dent is provided in the surface of the lowermost portion of a culture well.

### Best Mode for Carrying Out the Invention

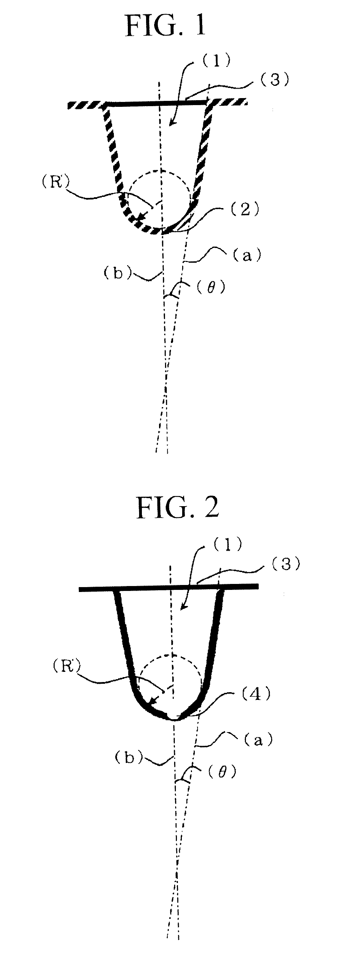

A key point of the culture vessel of the present invention is that if a multi-well plate, in which a lateral portion called a round bottom or a V-bottom is tapered toward the bottom surface and the tapered portion has a hemispheric or conical shape as shown in FIG. 1, is used, one cell aggregate is formed in each well in a uniform size, a cell aggregate of higher quality is formed, and an embryoid body can be formed. FIG. 1 shows an embodiment including two or more wells which have a bottom having a cross section that looks like approximately a U shape when being observed in a vertical direction and an opening having an approximately circular shape, in which at least a curved portion of the inner surface of the bottom, preferably, the entire inner surface of the wells has low cell adhesive properties, and the inner surface of the bottom of each well has a predetermined shape. If the well is formed such that the tapered portion has a conical shape or hemispherical shape, when cells are seeded into the well, cells gather in the bottom surface of the vessel due to the weight of the cell themselves, whereby a cell aggregate is easily formed.

The culture vessel of the present invention can be molded using a material made of a resin. If such a resin material is used, the culture vessel can be a disposable type, and various shapes can be easily molded. Examples of the resin material include polyolefin resins or cyclic polyolefin resins such as a polypropylene resin, a polyethylene resin, and an ethylene-propylene copolymer, polystyrene resins such as polystyrene and an acrylonitrile-butadiene-styrene resin, a polycarbonate resin, a polyethylene terephthalate resin, methacrylic resins such as a polymethyl methacrylate resin, a vinyl chloride resin, a polybutylene terephthalate resin, a polyarylate resin, a polysulfone resin, a polyether sulfone resin, a polyether ether ketone resin, a polyether imide resin, fluororesins such as a polytetrafluoroethylene resin, a polymethylpentene resin, an acrylic resin such as polyacrylonitrile, cellulose resins such as a propionate resin, and the like. Among these, in view of moldability and sterility required for culture vessels, a polystyrene resin is particularly preferable.

When the culture vessel of the present invention is produced from the above resin material, the vessel can be produced by, for example, injection molding, blow molding, or injection blow molding.

In the present invention, a curvature radius (R') of the inner surface of the bottom of each well is set to 3.5 mm or less and preferably set to 3.0 mm or less. In this manner, cells gather together at a sufficient density, whereby a cell aggregate of higher quality can be formed.

Moreover, the well shape of this vessel is further narrowed toward a bottom (2) compared to a traditional culture vessel in which a curvature radius (R') of the inner surface of the bottom exceeds 3.5 mm. Accordingly, when the medium is aspirated from the bottom (2) at the same height, the efficiency in replacing medium (ratio of the amount of medium that is aspirated and removed to the total amount of medium) becomes excellent, and this is one of the positive characteristics of the present invention.

In addition, if a curvature radius (R') is set to 1.0 mm or more, dead cells which are generated during culturing are not aggregated toward the bottom with an extremely high density, and the aggregation is excellently observed by an inverted microscope. Consequently, cells or an embryoid body in a well can be accurately observed.

Further, if the diameter of an opening (3) having an approximately circular shape as described above is set to 4.0 mm or more, operability in using a multi dispenser becomes excellent, and if it is set to 11.0 mm or less, multiple wells such as 48 or more wells can be provided to one culture vessel.

The volume of a well (1) is preferably from 80 µL to 500 µL. In this manner, it is possible to add a medium in an amount necessary and sufficient for forming one cell, for example, an embryoid body (EB) of a mouse ES cell or human ES cell, in each well.

The volume of the well (1) is more preferably from 80 µL to 200 µL. In this manner, the amount of the medium or reagent used can be reduced.

Each of the culture wells more preferably has the following structure. That is, as shown in FIG. 1, an angle (θ) at which a straight line (a) that extends from the ridge of the lateral surface of the culture well (1) beyond the bottom (2) crosses a straight line (b) that extends vertically from the center of the opening (3) beyond the bottom (2) of the culture well is preferably from 3° to 30°. In this manner, cells in the medium more easily gather in the bottom (2), and operation of a dispenser tip becomes easier in replacing the medium. The angle (θ) is more preferably from 5° to 15°. In this manner, a shape in which the bottom (2) having an approximately U-shape is more smoothly connected to the lateral surface is formed, whereby cells in the medium gather more easily, and an EB having a better shape can be formed.

In addition, as shown in FIG. 2, it is preferable that at least one dent (4) having a low cell adhesion surface is provided inside the surface of the lowermost portion of each well. In this structure, seeded cells efficiently gather in the dent of the lowermost portion of the well, and accordingly, a cell aggregate is excellently formed.

The volume of the dent (4) is not limited. However, if the volume is from 1.0 × 10⁻⁸ mL to 5.0 × 10⁻² mL, a cell aggregate is formed in the dent, and in replacing medium, it is possible to easily perform the operation for aspirating the old medium by using a dispenser without aspirating and collecting the cell aggregate in the dent. If the volume of the dent is from 2.0 × 10⁻⁷ mL to 2.0 × 10⁻² mL, this is preferable since it is possible to reduce the amount of the residual old medium during culture replacement without causing the cell aggregate to protrude from the dent even if the aggregate in the dent has grown. More preferably, if the volume of the dent is from 1.0 × 10⁻⁶ mL to 2.0 × 10⁻³ mL, medium replacement can be efficiently performed for an embryoid body of an embryonic stem cell having a size appropriate for the research on differentiation induction or an epithelial cell aggregate having a size appropriate for the research on stimulus-response.

The shape of the dent (4) is not particularly limited. However, the cross-sectional shape of the dent is preferably a shape that makes it easy for cells to gather in the bottom, such as an approximately U-shape, an approximately V-shape, or an approximately trapezoidal shape. If the shape is an approximately U-shape, this is particularly preferable since aggregating properties of cells become excellent, and positional stability of the formed aggregate becomes excellent.

The number of dents coming into contact with the inside of each well is not particularly limited, and one to multiple dents may be optionally formed. Here, the dent needs to be formed in the lowermost portion of the well such that the cells seeded in the well gather in the dent.

In the above respective embodiments of the present invention, the low cell adhesion surface of the well can be formed by the following low-or-non cell adhesion treatment. As the treatment for making a non cell adhesion, treatment for hydrophilizing the surface of a base material is preferable. The surface-hydrophilizing treatment mentioned herein includes the following treatment methods.
(1) Crosslinking and fixing water-soluble resin in the inner surface of a culture vessel
(2) Coating a surface with hydrophilic resin

First, the method (1) will be described in detail.

This method is characterized in that the formation of a water-soluble resin-covering layer in which a water-soluble resin covers the inner surface of a culture vessel and is crosslinked to form a water-soluble covering layer.

The water-soluble resin mentioned herein is hydrated by forming ionic bonds or hydrogen bonds with water molecules and consequently dissolves in water. In other words, the water-soluble resin is a resin that has ionic or polar side chains in a necessary and sufficient amount relative to the main chain in a molecule so as to dissolve in water. In addition, the water-soluble resin mentioned herein refers to a resin that can dissolve in an amount of 1.0 g or more in 100 g of water at 25°C.

Examples of the water-soluble resin include a saponified product of polyvinyl acetate, polyvinylpyrrolidone, polyethylene glycol, polyacrylamide, polymethacrylamide, polyhydroxyethyl methacrylate, polypentaerythritol triacrylate, polypentaerythritol tetraacrylate, polydiethylene glycol diacrylate, a copolymer of monomers constituting these, a copolymer of 2-methacryloyloxyethyl phosphorylcholine and other monomers (for example, butyl methacrylate), and the like. Among these, a structure including one or more kinds selected from a saponified product of polyvinyl acetate, polyvinylpyrrolidone, and polyethylene glycol and the above reactive groups is preferable. In this manner, it is possible to suppress a stimulus to various cells and to improve the formation speed and rate of a cell aggregate and the quality of the formed cell aggregate.

Herein, a saponified product of polyvinyl acetate refers to, for example, polyvinyl alcohol or a copolymer of vinyl alcohol and other compounds. The saponified product also includes, for example, vinyl alcohol and saponified products of modified vinyl acetate in which a reactive group such as a hydrophilic group, a hydrophobic group, an anion, a cation, an amide group, or an acetoacetyl group is modified.

When a polymer is used as the water-soluble resin, an average degree of polymerization thereof is preferably 100 to 10,000 and particularly preferably 200 to 5,000, though the degree is not particularly limited. If the average degree of polymerization is less than the above lower limit, it is difficult to evenly form a film on the surface of the cell culture vessel in some cases. If the average degree of polymerization exceeds the above upper limit, viscosity of the water-soluble resin is heightened, whereby operability deteriorates in some cases.

When the saponified product of polyvinyl acetate is used, a degree of saponification of the saponified product of polyvinyl acetate is not particularly limited. However, the degree of saponification is preferably from 20 mol% to 100 mol% and particularly preferably from 50 mol% to 95 mol% of the entire polyvinyl acetate.

As such a water-soluble resin, a resin containing a structural unit represented by, for example, the following Formula (Ia) or (Ib) is preferable. In this manner, it is possible to form a uniform film by using a wavelength of 300 nm to 500 nm which is practical, and to exceptionally improve a cell aggregate formation effect by reducing the amount of cells adhering to the vessel. 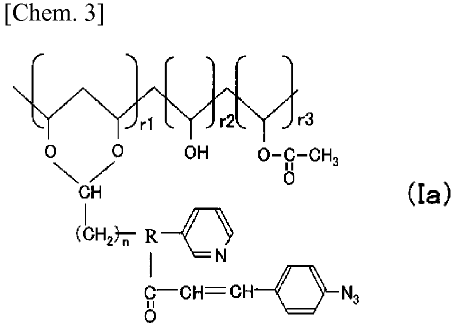 r1 = 1 to 1000, r2 = 40 to 4995, r3 = 0 to 4000, n = 1, 2 or 3, and R represents an alkyl group having carbonyl and amine. 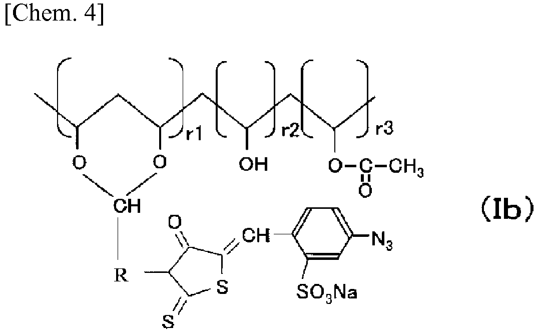 r1 = 1 to 1000, r2 = 40 to 4995, r3 = 0 to 4000, and R represents an alkyl group having carbonyl and amine.

In the water-soluble resin that is represented by Formula (Ia) or (Ib), R is not particularly limited as long as it is an alkyl group having carbonyl and amine. However, R is preferably a group represented by, for example, the following Formula (II). In this manner, the polar side chain described above can be easily synthesized.

When the cell culture vessel is impregnated with the water-soluble resin, it is preferable that the vessel is impregnated with the water-soluble resin which is in a state of being dissolved in a solvent. As the solvent used at that time, it is possible to use water or a mixture of water and an organic solvent to improve solubility.

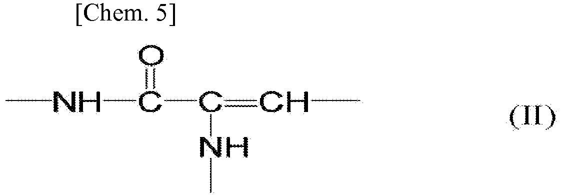

For example, when the water-soluble resin represented by Formula (Ia) or (Ib) is used, if an aqueous alcohol solution of 5% by volume to 40% by volume is used as the solvent, solubility of the water-soluble resin is increased, whereby a uniform covering layer can be formed.

The concentration of the water-soluble resin to be dissolved is preferably 0.01 % by weight to 30% by weight, and particularly preferably 0.1% by weight to 10% by weight.

Herein, if the concentration of the water-soluble resin is too low or too high, a uniform covering layer and a sufficient cell adhesion reducing effect are not obtained, and an excellent cell aggregate is not formed.

The thickness of the covering layer using the water-soluble resin is preferably from 100 nm to 5,000 nm, and more preferably from 150 nm to 1,000 nm. If the thickness of the covering layer is set to be the above lower limit or more, it is possible to further suppress a physical stimulus given to the cell from the base material. If the thickness is set to be the above upper limit or less, it is possible to inhibit cell adhesion caused via a protein by reducing the amount of protein absorbed into the covering layer, and accordingly, the formation rate of a cell aggregate can be further heightened.

As the method of covering the inner surface of the culture vessel with the water-soluble resin, for example, it is possible to use spin coating, dipping, or a method in which the water-soluble resin is dispensed to the inner surface of the culture vessel, and then the vessel is tilted to discharge the solution. The water-soluble resin is brought into contact with the inner surface of the culture vessel in this manner, and then the water-soluble resin solution remaining in the inner surface of the culture vessel is dried, whereby a water-soluble resin-covering layer can be formed.

The production method of the present invention is characterized by including a step of modifying into a water-insoluble cured film in which the water-soluble resin-covering layer is cured so as to be modified into a water-insoluble cured film layer, after the above step.

By modifying the water-soluble resin-covering layer into a water-insoluble cured film layer, it is possible to form a surface having ionic or polar side chains at a high density. When being brought into contact with a culture solution, the ionic or polar side chains formed on the surface are hydrated with water molecules by electrostatic interaction or hydrogen bonds, whereby the surface of the culture vessel practically becomes a hydrated layer containing dense water molecules. The hydrated layer suppresses the stimulus given to cells from the surface of the base material, whereby a cell aggregate of excellent quality is rapidly formed. In this manner, when being brought into contact with a culture solution, the covering layer of the water-soluble resin is prevented from being dissolved and liberated, and water resistance required for a culture vessel can be obtained.

The method of curing the water-soluble resin-covering layer is not particularly limited, and can be performed by introducing a water-soluble resin having a functional group for curing, for example, a radiation-reactive functional group, a photosensitive functional group, or a thermally reactive functional group, into the side chain of the water-soluble resin and curing the resin-introduced portion. Examples of the photosensitive functional group include a diazo group, an azido group, a cinnamoyl group, and the like, and examples of the thermally reactive and radiation-reactive functional groups include a vinyl group, an epoxy group, and the like. Among these, a water-soluble resin having a photosensitive functional group that makes it possible to rapidly perform curing treatment and can be cured with simple equipments is particularly preferable.

As the photosensitive functional group, a functional group having an azido group as shown in Formula (Ia) or (Ib) is particularly preferable. If this functional group is used, it is possible to react the functional group by using a wavelength of 230 nm to 500 nm which is practical and to improve film formability by excellent resolution. In this manner, by the step of forming a water-soluble resin-covering layer on the surface in advance and curing the covering layer to modify the covering layer into a water-insoluble cured film layer, a film layer having the same thickness as that of the above covering layer can be obtained.

One of the advantages obtained by using the water-soluble resin is that if the surface is washed with water after curing, the unreacted resin can be easily washed off. If effluent is detected due to for example poor curing reactivity, a washing step may be performed after curing, whereby the amount of the effluent is reduced, and a better formation rate of a cell aggregate can be obtained.

Next, the method (2) will be described.

The hydrophilic resin to be coated includes poly-2-hydroxyethyl methacrylate (poly-HEMA), a phosphorylcholine group-containing polymer compound, a polyethylene glycol chain-containing polymer compound, and the like, but the resin is not particularly limited to these.

For example, a 2% ethanol solution of poly-HEMA is dispensed in an amount of 100 µL into the vessel, and ethanol is evaporated, whereby a poly-HEMA layer can be formed on the surface of the vessel. If the vessel is washed with ultrapure water or a buffer solution after evaporation of ethanol, the surplus poly-HEMA molecules having not been adsorbed onto the surface of the vessel can be removed.

The effect of the method (2) is weaker than that of the method (1) since hydrophilization of the surface in the method (2) merely results in a phenomenon in which a polymer compound is adsorbed onto the surface of the vessel. However, simplicity is an advantage of the method.

Regarding sterilization as an essential condition of a culture vessel, ethylene oxide gas sterilization, dry-heat sterilization, steam sterilization, radiation sterilization, and the like are exemplified. Among these, radiation sterilization using γ-rays or electron beams is preferable. For mass production, γ-ray sterilization is particularly preferable in view of radiolucency.

The radiation absorbed dose is not particularly limited. However, when the absorbed dose is too low, sterility is not secured, and if it is too high, the cell culture vessel and the covering layer deteriorate in some cases.

The culture vessel prepared as above has the following characteristics.

Since the surface of the base material is performed non cell adhesion treatment and has a conical or hemispherical shape, cells easily gather at the bottom, and a cell aggregate is formed easily.

Moreover, the formed cell aggregate in the plate can be used as is for fluorescence or luminescence assay.

### Examples

Hereinafter, the present invention will be described in detail based on examples, but the present invention is not limited thereto.

### (Examples 1 to 5)

By using a polystyrene resin (manufactured by PS Japan Corporation, HF77) as a resin material, a 96-well multi-well plate was molded by injection molding. The shape of each well was as described in FIG. 1, and in each of Examples 1 to 5, the angle of the tapered portion was set to 3°, 5°, 10°, 20°, and 30°.

The obtained plate was subjected to plasma treatment (oxygen plasma, 10 minutes) by using a plasma treatment device (manufactured by BRANSON/IPC, SERIES 7000), and as pretreatment, wettability was imparted to the plate surface.

Thereafter, as a water-soluble resin, polyvinyl alcohol (manufactured by Toyo Gosei Co., Ltd., AWP: a compound represented by Formula (Ia) (an average degree of polymerization of the water-soluble resin: 1600, proportion of an introduced photosensitive group: 0.65 mol%)) having an azido group on the side chain was dissolved in 25% by volume of aqueous ethanol solution in a polypropylene vessel protected from light by using a colored resin, thereby preparing 0.3% by weight of a solution.

In the above plate, 100 µL of the above water-soluble resin solution was added to each well by using an automatic dispenser (manufactured by BIOTEC Co., Ltd., Auto Sera Washer AMW-96SX), and the plate was impregnated with the solution for 1 minute. Subsequently, the plate was turned over to sufficiently discard the solution and then subjected to primary drying for 17 hours at 25°C. Then the plate was irradiated with UV light of 250 nm for 30 seconds at 2.0 mW/cm² by using a UV lamp, thereby curing the water-soluble resin. Thereafter, the plate was washed three times with ultrapure water and dried, and then irradiated with γ-rays at an absorbed dose of 5.8 kGy (RADIA INDUSTRY CO., LTD.), thereby obtaining the culture vessel (plate) of the present invention.

### (Comparative Example 1)

A culture vessel (plate) was obtained in the same manner as in Example 1, except that the step of imparting hydrophilicity by plasma treatment to the step of impregnating the plate with a water-soluble resin, and the respective steps including the step of impregnating the plate with a water-soluble resin, curing, washing, and drying in Example 1 were not performed.

### (Comparative Example 2)

A culture vessel (plate) was obtained in the same manner as in Example 1, except that the angle of the tapered portion of each well of a 96-well multi plate was set to 2°.

### (Comparative Example 3)

A culture vessel (plate) was obtained in the same manner as in Example 1, except that the angle of the tapered portion of each well of a 96-well multi plate was set to 35°.

### (Comparative Example 4)

A culture vessel (plate) was obtained in the same manner as in Example 1, except that a 96-well multi plate (manufactured by Sumitomo Bakelite Co., Ltd., MS-8096F) having wells with a planar bottom was used.

The culture vessels obtained in Examples 1 to 5 and Comparative Examples 1 to 4 were respectively evaluated as below.

### (1) Formation of embryoid body by using mouse ES cells

A cell suspension obtained by dispersing mouse ES cells in a culture solution (Dulbecco's modified MEM + 15% fetal bovine serum) at a concentration of 7,500 cells/mL was prepared and dispensed to 96 wells of the above plate at 100 µL/well, followed by culturing for 5 days in a 5% CO₂ atmosphere at 37°C.

After 5 days, each well was observed with a microscope to examine whether or not an embryoid body was formed.

### (2) Examination of differentiation of mouse ES cell embryoid body into myocardium

Thereafter, the cultured embryoid body was transferred to a 24-well multi plate (manufactured by Sumitomo Bakelite Co., Ltd., MS-80240) at one aggregate/well, and then a culture solution (Dulbecco's modified MEM + 15% fetal bovine serum) was dispensed to the wells at 500µL/well, followed by culturing for 5 days in a 5% CO₂ atmosphere. After 5 days, each well was observed with a microscope to examine whether or not the aggregates have pulsation peculiar to myocardial cells.

As a result, it was revealed that in all of Examples 1 to 5 using the vessel of the present invention, a single spherical embryoid body was formed in all wells, as shown in Table 1. Moreover, in all of the obtained embryoid bodies, pulsation peculiar to myocardial cells was observed.

On the other hand, in Comparative Example 1, cells in all wells underwent adhesion and extension, an embryoid body was not formed, and pulsation peculiar to myocardial cells could not be observed at all.

In Comparative Example 2, the formation of an embryoid body was observed in all wells. However, dead cells were precipitated around the embryoid body, and the proportion of wells in which pulsation peculiar to myocardial cells was observed was greatly reduced to 11.5%.

In Comparative Example 3, the formation of an embryoid body was observed in all wells, but the formation of plural embryoid bodies was observed in half or more of the wells. A proportion of wells in which pulsation peculiar to myocardial cells was observed was 47.9% which was half or less of the wells.

Moreover, in Comparative Example 4, the formation of an embryoid body was observed in all wells, but the formation of plural embryoid bodies was observed in 80% or more of wells. The proportion of wells in which pulsation peculiar to myocardial cells was observed was greatly reduced to 27.1 %.

**[Table 1]**

| | Form of aggregate | Formation rate of embryoid body | Formation rate of plural embryoid bodies | Proportion of wells in which pulsation peculiar to myocardial cells was observed |
|---|---|---|---|---|
| Examples 1 to 5 | Single embryoid body | 100% | 0% | 100% |
| Comparative Example 1 | Cells underwent adhesion and extension. Embryoid body was not formed. | 0% | 0% | Embryoid body was not formed. |
| Comparative Example 2 | Dead cells were precipitated around embryoid body. | 100% | 0% | 11.5% |
| Comparative Example 3 | Plural embryoid bodies were formed. | 100% | 59.4% | 47.9% |
| Comparative Example 4 | Plural embryoid bodies were formed. | 100% | 82.2% | 27.1% |

### Industrial Applicability

If the culture vessel for forming a cell aggregate of the present invention is used, cells can be cultured while maintaining their function. Moreover, the culture vessel can be used for assay using functional cells. Accordingly, the present invention is extremely useful industrially.

## Claims

1. A culture vessel for forming an embryoid body that has two or more wells, wherein the wells have a bottom having a cross section that looks like an approximately U shape when being observed in a vertical direction and an opening having an approximately circular shape, at least a curved portion of an inner surface of the bottom has low cell adhesive properties, a curvature radius (R') of the inner surface of the bottom is from 1.0 mm to 3.5 mm, and the low cell adhesion surface is formed by being performed low-or-non cell adhesion treatment with a water-soluble resin.

2. The culture vessel for forming an embryoid body according to Claim 1,
wherein the water-soluble resin is a compound represented by the following Formula (Ia) or (Ib), 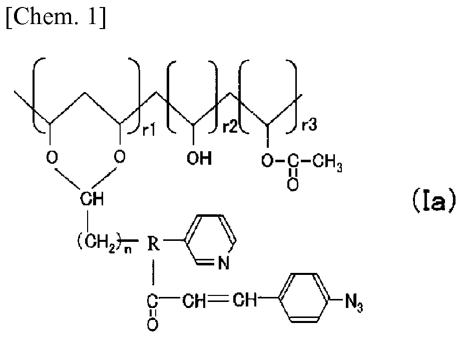 wherein r1 = 1 to 1000, r2 = 40 to 4995, r3 = 0 to 4000, n = 1, 2 or 3, and R represents an alkyl group having carbonyl and amine, 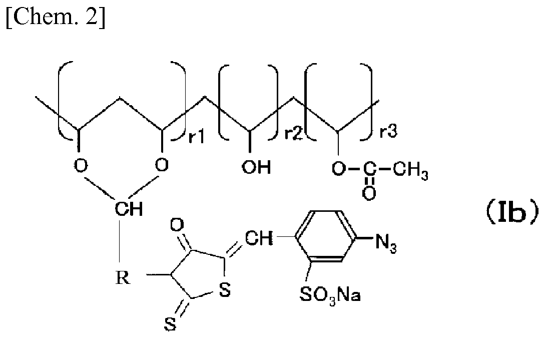 wherein r1 = 1 to 1000, r2 = 40 to 4995, r3 = 0 to 4000, and R represents an alkyl group having carbonyl and amine.

3. The culture vessel for forming an embryoid body according to Claim 1 or 2,
wherein at least one dent having a low cell adhesion surface is provided inside the surface of the lowermost portion of the well.

4. The culture vessel for forming an embryoid body according to any one of Claims 1 to 3,
wherein an entire inner surface of each well has low cell adhesive properties.
